Europäisches Patentamt

European Patent Office

Office européen des brevets

(11) Publication number: **0 257 777 B1**

## EUROPEAN PATENT SPECIFICATION

(45) Date of publication of patent specification: **31.03.93**  (51) Int. Cl.⁵: **A61C  9/00**

(21) Application number: **87306391.1**

(22) Date of filing: **20.07.87**

(54) **Method of making dental impressions.**

(30) Priority: **25.08.86 US 900110**

(43) Date of publication of application:
**02.03.88 Bulletin  88/09**

(45) Publication of the grant of the patent:
**31.03.93 Bulletin  93/13**

(84) Designated Contracting States:
**CH DE FR GB IT LI SE**

(56) References cited:
**EP-A- 0 130 598
EP-A- 0 173 085
DE-A- 2 512 443
GB-A- 1 521 446
US-A- 4 468 202**

(73) Proprietor: **KERR MANUFACTURING COM-
PANY
28200 Wick Road, Box 455
Romulus, Michigan 48174(US)**

(72) Inventor: **Hamilton, James Crawford
1906 Mershon Drive
Ann Arbor Michigan(US)**
Inventor: **Waller, Duncan Ewan
5504 New Meadow Drive
Ypsilanti Michigan(US)**
Inventor: **Semkow, Christina Lynn
4131 Green Meadows Boulevard No. 111
Ypsilanti Michigan(US)**

(74) Representative: **Ackroyd, Robert et al
W.P. THOMPSON & CO. Eastcheap House
Central Approach
Letchworth, Hertfordshire SG6 3DS (GB)**

**Description**

This invention relates to a method of forming an intra-oral impression of tooth structure and soft tissue.

The invention overcomes two common difficulties associated with the use of light-curable impression material of the prior art. The first difficulty is the problem associated with attempting to cure the entire mass of the light-curable impression material at one time including the material underneath the gingival margin. The second problem is that associated with the light-curable material which is expelled from the tray during the seating of the tray and which cannot be exposed to the light source.

One specific prior art document is US-A-4468202 (Cohen) which describes a method of obtaining a dental impression in which successive durable elastomeric materials are applied to the oral cavity. Each material may be either photocurable or chemically curable.

It is an object of the present invention to overcome the above problems associated with the prior art, in addition to providing a number of unique advantages relating to ease and convenience of handling impression material and better and more efficient curing.

The present invention provides a method of forming a dental impression, the method being as defined in claim 1.

The invention is concerned with facilitating the forming of an intra-oral impression of tooth structure and soft tissue. The forming procedure is preferably carried out as follows. The mouth is prepared in the same manner as is currently done in the dental art to take an impression. Instead of injecting a conventional chemically-activated, low viscosity impression material, a light-activated, low viscosity material is first used. When the parts of the oral cavity that are of interest, such as the prepared teeth, are covered with the light-curable impression material, the material is then set with visible light energy in a suitable range, preferably from 370 to 550 nanometers. A suitable light source comprises the Kerr Command Light™, sold by Kerr Manufacturing Company. As the light-curable material is being set, a chemically-curing material is mixed (either a base-catalyst or a base-accelerator system) which is thixotropic. This material is placed in a conventional or custom impression tray such as a Kerr Manufacturing Company Kwik Tray™ or Formatray™ and is seated in the mouth covering the at least partially set, light-curable impression material and the surrounding oral tissues. When the chemically-curing impression material has set, the tray containing the light-cured impression material along with the chemically-cured impression material is removed from the mouth as one unit.

The following light-curable impression materials are used in forming the initial impression:

Adducts of long chain aliphatic polyester polyols with aliphatic diisocyanates, endcapped with hydroxy functional acrylates or methacrylates, and modified by the incorporation of photosensitizing components, plasticizers, fillers,pigments, tack reducing additives and stabilizers, such as described for example in US-A-4761136.

A specific suitable light-curable impression material comprises the following which is disclosed in Example 2 of the above-mentioned patent.

| Rucoflex S-1011-55* | 456.0 parts (.456 equiv.) |
|---|---|
| 1,6 Hexanediisocyanate | 63.8 parts (.760 equiv.) |
| 2-Hydroxypropylmethacrylate | 43.8 parts (.304 equiv.) |
| Santicizer 261** | 338.0 parts |
| Dimethylaminobenzaldehyde | 4.6 parts |
| Aerosil R-972*** | 19.0 parts |
| Quso**** | 68.4 parts |
| Dibutyltindilaurate (T-12) | 0.4 parts |
| Irganox 1010***** | 0.2 parts |
| Modaflow****** | 0.9 parts |
| Camphroquinone | 0.6 parts |
| Meteor Cobalt Blue (Harshaw Chemical) | 2.0 parts |

* A polymeric 2,2'-oxybis (ethanol) hexanedioic acid ester, $(C_6H_{10}O_4C_4H_{10}O_3)$

** Octyl benzyl phthalate available from Monsanto Chemical Company

*** A hydrophobic submicron synthetic amorphous precipitated silica available from DeGussa

**** Quso WR 55 from PQ Corporation, a synthetic amorphous precipitated silica

***** Tetrakis (Methylene (3,5-di-tert-butyl-4-hydroxy hydrocinnamate)) methane

****** Ethyl acrylate and 2-ethyl hexyl acrylate copolymer

The following chemically-curing impression materials are used in forming the second part of the composite impression of the present invention:

Two-component acrylic functional materials having the following general composition and preferably used in approximately 10:1 base to catalyst ratio.

3

| BASE PASTE | Wt. % |
|---|---|
| Difunctional urethane methacrylate prepolymer | 30 to 60 |
| Diluent acrylic functional monomer | 3 to 15 |
| Acrylic functional tertiary amine | 0.2 to 5.0 |
| Filler(s) | 20 to 70 |
| Pigment(s) | 0.1 to 3.0 |

Example

| | Wt. % |
|---|---|
| Polyesterpolyolhexamethylenediisocyanatehydroxypropyl-methacrylate adduct | 40.0 |
| Polypropyleneglycoldimethacrylate | 8.5 |
| Dimethylaminoethylmethacrylate | 1.0 |
| Micronized silica filler | 50.0 |
| FD&C Blue #2 Lake pigment | 0.5 |
| | 100.0 |

| CATALYST PASTE | Wt. % |
|---|---|
| Acrylic functional crosslinking monomer | 30 to 60 |
| Acyl peroxide | 0.5 to 10.0 |
| Filler(s) | 20 to 70 |
| Inhibitor/stabilizer | 0.01 to 0.5 |

Example

| | Wt. % |
|---|---|
| Ethoxylated bisphenol A dimethacrylate | 50.0 |
| Benzoyl peroxide | 4.9 |
| Micronized silica filler | 45.0 |
| Inhibitor(s) (4-methoxyphenol/Irganox 1010 blend) | 0.1 |
| | 100.0 |

The two-component chemically-curing material is intended to bond to a single-component, visible light activated, acrylic functional impression material in a two-step technique, wherein a light-bodied, visible light activated impression material is first injected around the dentition and cured, after which a mix of the two-component impression material is placed, preferably in a tray, over the cured light-bodied material and allowed to polymerise and bond to it, to provide dimensional stability. The attachment between the light-cured impression material and the chemically-curing impression material is essentially by chemical bonding between the two.

The two-component acrylic functional impression material is chemically cured by free radical polymerisation, the free radicals being generated by the interaction of the acyl peroxide in one component with the tertiary amine in the other component by the classic Swenck redox mechanism.

Since the single-component, light-activated impression material cures with a slightly air-inhibited surface, the two-component material placed over it acts as an air excluder and enables a strong interfacial bond to develop by copolymerisation as the two-component material polymerises.

The acyl peroxide catalyst contains an acrylic functional crosslinking monomer plus inhibitor(s) to control the rate of reaction and suppress autopolymerisation tendencies of the catalyst paste during shelf life.

The following example specifically illustrates one embodiment of the present invention.

4

EXAMPLE

The gingiva around the tooth being impressed is retracted using a gingival retraction cord. After sufficient time has elapsed for the gingiva to retract, the cord is removed and the light-curable impression material described above, pre-packaged in a light-proof syringe, is injected into the sulcus around the tooth. This material is then cured with visible light from a Kerr Command curing unit using a standard 5 mm light guide. While the light-curable material is being injected around the tooth, the base and catalyst of the chemically-activated impression material described in the above examples are mixed together and loaded into a Kerr Kwik-Tray™. After the light-curable material has set, the mixed chemically-activated material is placed directly over the at least partially set light-curable material. When the chemically-activated material has set, the tray is removed from the mouth, taking with it the entrapped chemically-bonded, light-cured impression material.

An impression of the prepared tooth is captured in the light-cured material while adjacent teeth, as well as surrounding tissue, are captured in the chemically-activated material.

This invention overcomes two difficulties associated with light-cured impression materials. The first is curing all the mass of the light-curable impression material at one time, including the material underneath the gingival margin. The second has to do with light curing the material that is expelled from the tray during the seating of the tray. The present invention provides a number of additional advantages. For example, the dentist will not need to mix a low viscosity material; thus, the light-cured material will be free from voids, such as are usually encountered when air is accidentally mixed into the material during the mixing of the base and catalyst portions of an impression material system. This material can be prepackaged in a light-proof syringe, with light-proof disposable tips. The dentist can therefore inject the material directly into the mouth at the exact location of interest. The dentist can take as much or as little time as necessary to inject the light-bodied material and then achieve setting on command by just exposing that material to the visible light source. Because only a thin layer of material needs to be cured in this situation, light from a regular dental composite restorative curing unit such as a Kerr Command Light can be used to polymerise the impression material. Specially designed curing wand tips can also be used. These take the form of "U" shaped channel members so that the light-curable impression material is cured from three sides at the same time. The open portion of the "U" channel is placed over the tooth covered with light-curable impression material. By using a chemically-curing product to take an impression over the light-cured product, the concern about uncured material that is pushed beyond the edge of the tray and underneath the gingival margin is obviated.

**Claims**

1. A method of forming a dental impression which comprises:
   (a) applying a light-curable impression material to a portion of an oral cavity of interest to form an initial impression and exposing the impression material to a source of activating light to at least partially set the impression material;
   (b) applying a chemically-curing impression material over the light-cured impression material and allowing the chemically-curing impression material to set and bond with the light-curable material; and
   (c) removing the resulting impression from said oral cavity, wherein
   the light-curable impression material comprises an adduct of a long chain aliphatic polyester polyol with an aliphatic diisocyanate, endcapped with a hydroxy functional acrylate or methacrylate, and modified by the incorporation of photosensitizing components, plasticizers, fillers, pigments, tack reducing additives and stabilizers, and the chemically-curing impression material comprises a base paste and a catalyst paste as hereinafter defined, mixed in a suitable ratio:

| BASE PASTE | Wt % |
|---|---|
| Difunctional urethane methacrylate prepolymer | 30 to 60 |
| Diluent acrylic functional monomer | 3 to 15 |
| Acrylic functional tertiary amine | 0.2 to 5.0 |
| Filler(s) | 20 to 70 |
| Pigment(s) | 0.1 to 3.0 |

| CATALYST PASTE | Wt% |
|---|---|
| Acrylic functional crosslinking monomer | 30 to 60 |
| Acyl peroxide | 0.5 to 10.0 |
| Filler(s) | 20 to 70 |
| Inhibitor/stabilizer | 0.01 to 0.5 |

**2.** A method according to claim 1, in which the chemically-curing impression material is applied by being placed in a dental tray which is then seated over the light-curable material.

**3.** A method according to claim 1 or 2, in which the light-curable impression material is exposed to activating light in the range of from 370 to 550 nm.

**Patentansprüche**

**1.** Verfahren zur Herstellung eines Gebißabdrucks, wobei
(a) eine durch Licht härtbare Abdruckmasse in einen Bereich der interessierenden Mundhöhle eingebracht wird, um einen anfänglichen Abdruck zu bilden, und die Abdruckmasse einer Quelle für aktivierendes Licht ausgesetzt wird, um die Abdruckmasse mindestens teilweise auszuhärten,
(b) eine chemisch härtende Abdruckmasse über der durch Licht gehärteten Abdruckmasse aufgetragen wird, und die chemisch härtende Abdruckmasse aushärtet und mit dem durch Licht härtbaren Material eine Bindung eingeht, und
(c) der resultierende Abdruck aus der Mundhöhle entfernt wird, wobei
die durch Licht härtbare Abdruckmasse ein Addukt eines langkettigen aliphatischen Polyesterpolyols mit einem aliphatischen Diisocyanat enthält, das mit einem Hydroxy-funktionellen Acrylat oder Methacrylat verbunden ist, und modifiziert ist durch photoempfindlich machende Komponenten, Weichmacher, Füllstoffe, Pigmente, Haftung verringernde Zusätze und Stabilisatoren, und wobei die chemisch härtende Abdruckmasse die folgende mit einer Katalysatorpaste in einem geeigneten Verhältnis gemischte Basispaste enthält:

| BASISPASTE | Gewichtsprozent |
|---|---|
| Difunktionelles Urethanmethacrylat-Präpolymeres | 30 bis 60 |
| Verdünnungsmittel Acryl-funktionelles Monomeres | 3 bis 15 |
| Acryl-funktionelles tertiäres Amin | 0,2 bis 5,0 |
| Füllstoff(e) | 20 bis 70 |
| Pigment(e) | 0,1 bis 3,0 |

| KATALYSATORPASTE | |
|---|---|
| Acryl-funktionelles vernetzendes Monomeres | 30 bis 60 |
| Acylperoxid | 0,5 bis 10,0 |
| Füllstoff(e) | 20 bis 70 |
| Inhibitor/Stabilisator | 0,01 bis 0,5. |

**2.** Verfahren nach Anspruch 1, bei dem die chemisch härtende Abdruckmasse mit einer Schale für zahnärztliche Zwecke aufgebracht wird, die über dem durch Licht härtbaren Material aufgesetzt wird.

**3.** Verfahren nach Anspruch 1 oder 2, bei dem die durch Licht härtbare Abdruckmasse mit aktivierendem Licht in einem Wellenlängenbereich zwischen 370 und 55 nm belichtet wird.

## Revendications

1. Procédé de réalisation d'une prise d'empreinte dentaire, qui comprend :

   (a) l'application d'une matière, photodurcissable pour prise d'empreinte, sur une partie d'une cavité buccale intéressante, pour former une empreinte initiale et l'exposition de la matière pour empreinte à une source de lumière d'activation afin de faire durcir au moins partiellement la matière pour empreinte ;

   (b) l'application d'une matière, capable d'un durcissement chimique, pour formation d'empreinte, application réalisée par dessus la matière photodurcie pour empreinte, et le fait de laisser cette matière, capable de durcir par voie chimique pour former une empreinte, durcir et se lier à la matière photodurcissable ; et

   (c) l'enlèvement de l'empreinte résultante de ladite cavité buccale, procédé dans lequel

   la matière photodurcissable pour prise d'empreinte comprend un produit d'addition d'un polyester polyol aliphatique à longue chaîne avec un diisocyanate aliphatique, à extrémités coiffées par un acrylate ou méthacrylate comportant un groupe hydroxyle fonctionnel, et modifié par l'incorporation de constituants de photosensibilisation, de plastifiants, de charges, de pigments, d'additifs diminuant la pégosité et de stabilisants, et la matière capable de durcir par voie chimique pour réalisation d'une empreinte comprend une pâte de base et une pâte de catalyseur selon la définition ci-après, qui sont mélangées selon un rapport convenable :

| PATE DE BASE | % en poids |
|---|---|
| Prépolymère d'uréthanne-méthacrylate difonctionnel | 30 à 60 |
| Diluant : monomère à groupe acrylique fonctionnel | 3 à 15 |
| Amine tertiaire à groupe acrylique fonctionnel | 0,2 à 5,0 |
| Charge(s) | 20 à 70 |
| Pigment(s) | 0,1 à 3,0 |

| PATE DE CATALYSEUR | % en poids |
|---|---|
| Monomère à groupe acrylique fonctionnel, agent de réticulation | 30 à 60 |
| Peroxyde d'acyle | 0,5 à 10,0 |
| Charge(s) | 20 à 70 |
| Inhibiteur/stabilisant | 0,01 à 0,5 |

2. Procédé selon la revendication 1, dans lequel la matière capable de durcir par voie chimique, pour prise et formation d'une empreinte, est appliquée par son placement dans un porte-empreinte dentaire qui est ensuite mis en place par-dessus la matière photodurcissable.

3. Procédé selon la revendication 1 ou 2, dans lequel la matière photodurcissable pour prise d'empreinte est exposée à une lumière d'activation dont la longueur d'onde se situe entre 370 et 550 nm.